(19) Europäisches Patentamt
European Patent Office
Office européen des brevets

(11) Veröffentlichungsnummer : **0 021 012**
**B1**

(12) # EUROPÄISCHE PATENTSCHRIFT

(45) Veröffentlichungstag der Patentschrift : **16.02.83**

(51) Int. Cl.$^3$ : **C 07 C125/065**

(21) Anmeldenummer : **80102716.0**

(22) Anmeldetag : **16.05.80**

(54) **Verfahren zur Herstellung von p-substituierten, aromatischen Carbaminsäureestern.**

(30) Priorität : **23.06.79 DE 2925480**

(43) Veröffentlichungstag der Anmeldung : **07.01.81 Patentblatt 81/01**

(45) Bekanntmachung des Hinweises auf die Patenterteilung : **16.02.83 Patentblatt 83/07**

(84) Benannte Vertragsstaaten : **BE CH DE FR GB LI NL**

(56) Entgegenhaltungen :
**US A 2 695 913**
**H.R. CHRISTEN « Grundlagen der organischen Chemie » 1. Auflage 1970, VERLAG SAUERLÄNDER, Diesterweg-Salle, Aarau, Frankfurt**

**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Merger, Franz, Dr.**
**Max-Slevogt-Strasse 25**
**D-6710 Frankenthal (DE)**
Erfinder : **Nestler, Gerhard, Dr.**
**Rubensstrasse 25**
**D-6700 Ludwigshafen (DE)**

**EP 0 021 012 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von p-substituierten, aromatischen Carbaminsäureestern

Die Erfindung betrifft ein neues Verfahren zur Herstellung von p-substituierten, aromatischen Carbaminsäureestern durch Umsetzung von aromatischen Carbaminsäureestern mit Olefinen in Gegenwart anorganischer Säuren oder Sulfonsäuren.

Es ist aus Houben-Weyl, Methoden der Organischen Chemie, Band 11/1, Seiten 137 bis 145, bekannt, aromatische Carbaminsäureester durch Umsetzung der entsprechenden aromatischen Amine mit Chlorameisensäureester in Gegenwart eines saurebindenden Mittels oder durch Umsetzung der entsprechenden Arylisocyanate mit Alkoholen herzustellen. Der wesentliche Nachteil dieser Verfahren besteht darin, daß sie von den bekanntlich nur schwer zugänglichen para-kernalkylierten Anilinen ausgehen. US 2 695 913 beschreibt die Herstellung von speziellen Alkylarylurethanen aus Alkylarylisocyanaten und Polyoxyverbindungen z. B. Polyoxyalkylenglykolen. Die benötigten Alkylarylisocyanate werden dabei nach bekannten Methoden, nämlich durch säurekatalysierte Alkylierung von Aromaten, Nitrierung mit Salpetersäure/Schwefelsäure, katalytische Reduktion mit Wasserstoff und anschließender Phosgenierung der Amine hergestellt. Alle diese Verfahren befriedigen mit Bezug auf Ausbeute und einfachen und wirtschaftlichen Betrieb nicht.

Es wurde nun gefunden, daß man p-substituierte, aromatische Carbaminsäureester der Formel

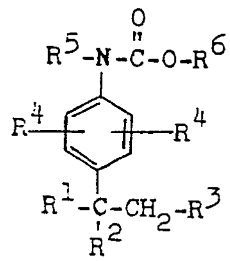

I

worin die einzelnen Reste $R^1$, $R^2$, $R^3$, $R^4$, $R^5$ und $R^6$ gleich oder verschieden sein können und jeweils einen aliphatischen Rest bedeuten, $R^1$, $R^2$ und $R^3$ auch jeweils einen WB/BL cycloaliphatischen, araliphatischen oder aromatischen Rest bezeichnen, jeweils 2 der Reste $R^1$, $R^2$ und $R^3$ auch zusammen mit den benachbarten Kohlenstoffatomen für Glieder eines alicyclischen Ringes oder $R^1$, $R^2$ und $R^3$ auch zusammen mit den benachbarten Kohlenstoffatomen für einen bicyclischen Rest stehen, $R^2$, $R^3$ und $R^5$ auch jeweils ein Wasserstoffatom bezeichnen, $R^4$ auch ein Wasserstoffatom oder ein Halogenatom bedeutet, $R^6$ auch einen cycloaliphatischen Rest bezeichnet, vorteilhaft erhält, wenn man aromatische Carbaminsäureester der Formel

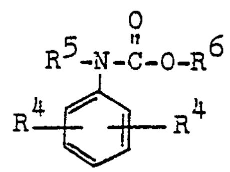

II

worin $R^4$, $R^5$ und $R^6$ die vorgenannte Bedeutung besitzen, mit Olefinen der Formel

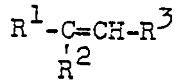

III

worin $R^1$, $R^2$ und $R^3$ die vorgenannte Bedeutung besitzen, in Gegenwart von anorganischen Säuren oder Sulfonsäuren umsetzt.

Die Umsetzung kann für den Fall der Verwendung des N-Phenylcarbaminsäureäthylesters und Isobuten durch die folgenden Formeln wiedergegeben werden :

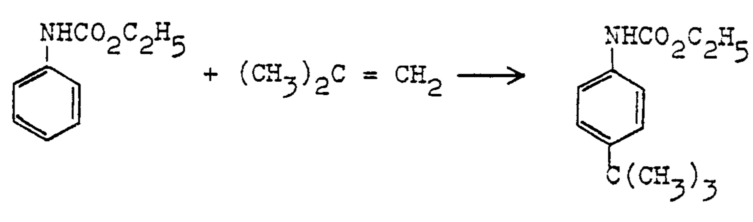

Im Vergleich zu den bekannten Verfahren liefert das Verfahren nach der Erfindung überraschend auf einfacherem und wirtschaftlicherem Wege eine große Zahl von p-substituierten, aromatischen Carbaminsäureestern in besserer Raum-Zeit-Ausbeute und Reinheit, gerade auch im großtechnischen Maßstab und im kontinuierlichen Betrieb. Es war überraschend, daß eine p-Substituierung eines N-Arylcarbaminsäureesters bereits mit katalytischen Mengen einer Säure in guter Ausbeute durchgeführt werden konnte. Im Hinblick auf die Lehre, daß Carbaminsäureester in Gegenwart von Säuren gespalten werden (Houben-Weyl, Band 11/1, Seiten 948), war außerdem nicht zu erwarten, daß die Substituierung in Gegenwart von Säuren ohne Bildung heterogener Gemische mit wesentlichen Mengen an Nebenprodukten durchführbar ist.

Das Olefin III kann mit dem aromatischen Carbaminsäureester II in stöchiometrischer Menge, im Überschuß oder Unterschuß, vorzugsweise in einem Verhältnis von 0,3 bis 5, insbesondere 0,5 bis 4 Mol Olefin je Mol Carbaminsäureester II umgesetzt werden. Bevorzugte aromatische Carbaminsäureester II, Olefine III, und dementsprechend bevorzugte aromatische Carbaminsäuren I sind solche, in deren Formeln die einzelnen Reste $R^1$, $R^2$, $R^3$, $R^4$, $R^5$ und $R^6$ gleich oder verschieden sein können und jeweils einen Alkylrest mit 1 bis 10, insbesondere 1 bis 4 Kohlenstoffatomen bedeuten, $R^1$, $R^2$ und $R^3$ auch jeweils einen Cycloalkylrest mit 5 bis 8 Kohlenstoffatomen, einen Aralkylrest oder Alkylarylrest mit 7 bis 12 Kohlenstoffatomen oder einen Phenylrest bezeichnen, jeweils 2 der Reste $R^1$, $R^2$ und $R^3$ auch zusammen mit den benachbarten Kohlenstoffatomen für Glieder eines alicyclischen Ringes mit 5 bis 8 Kohlenstoffatomen oder $R^1$, $R^2$ und $R^3$ auch zusammen mit den benachbarten Kohlenstoffatomen im Ausgangsstoff II für Norbornen und in diesem Fall im Endstoff I für einen Norbornylrest stehen, $R^2$, $R^3$ und $R^5$ auch jeweils ein Wasserstoffatom bezeichnen, $R^4$ auch ein Wasserstoffatom oder ein Bromatom oder ein Chloratom bedeutet, $R^6$ auch einen Cycloalkylrest mit 5 bis 8 Kohlenstoffatomen bezeichnet. Die vorgenannten Reste und Ringe können noch durch unter den Reaktionsbedingungen inerte Gruppen, z. B. Alkylgruppen oder Alkoxygruppen mit 1 bis 5 Kohlenstoffatomen, den Phenylrest substituierende Chlor- oder Bromatome, substituiert sein.

Bevorzugt verwendet man verzweigte Alkene. Es können im Gegensatz zu den bekannten Verfahren vorteilhaft auch Gemische von Alkenen und gegebenenfalls von Alkenen mit Alkanen verwendet werden, wie sie z.B. bei der Crackung oder Dehydrierung von Kohlenwasserstoffen, z. B. Erdöl, oder der Oligomerisierung von Olefinen, insbesondere von Isobutylen, Propylen oder n-Buten, oder der Kohlenoxidhydrierung entstehen. Anstelle der Olefine können auch unter den Reaktionsbedingungen olefinbildende Verbindungen, beispielsweise Äther wie Methyl-tert.-butyläther, Ester wie tert.-Butylacetat oder Alkohole wie tert.-Amylalkohol oder tert.-Butanol eingesetzt werden.

Es können z. B. folgende Olefine als Ausgangsstoffe II verwendet werden : n-Penten-(1), n-Hexen-(1), n-Hepten-(1), n-Octen-(1), n-Nonen-(1), n-Decen-(1), n-Undecen-(1), n-Dodecen-(1), Propylen-(1), n-Buten-(1) ; vorgenannte Alkene, die in 2-Stellung oder 3-Stellung oder 4-Stellung durch die Methyl-, Äthyl-, n-Propyl-, Isopropyl-, n-Butyl-, Isobutyl-, sek.-Butyl-, tert.-Butyl-Gruppe substituiert sind ; 2,3-Dimethyl-n-buten, 3,3-Dimethyl-n-buten, 2-Methyl-3-äthylpenten, 3-Methyl-3-äthylpenten, 2,3,3-Trimethylhepten, 2,4,4-Trimethylpenten, 2,3,3-Trimethylpenten, 2,3,4-Trimethylpenten ; analoge Alkene, deren Doppelbindung in 2-Stellung oder in 3-Stellung im Molekül liegen ; verzweigte Alkene, wie sie in Gestalt von Gemischen bei der Dimerisierung von Isobutylen oder n-Buten (Octene) oder Trimerisierung von Isobutylen oder n-Buten (Dodecene) oder Propylen (Nonene) bzw. Tetramerisierung von Propylen (Dodecene) anfallen ; o-Methylstyrol, m-Methylstyrol, p-Methylstyrol, 3,5-Dimethylstyrol, o-Äthylstyrol, m-Äthylstyrol, p-Äthylstyrol, 4-Chlorstyrol ; Cyclohepten, α-Methylstyrol, Norbornen, Cyclohexen, 1-Methylcyclohexen.

Bevorzugt sind : Propylen, Isobutylen, Styrol, n-Octene, n-Nonene, n-Decene und n-Dodecene, 2,3-Dimethylbuten-1, 2-Methylbuten-1,2-Methylbuten-2, n-Buten, 2-Äthylbuten-1, Cyclopenten, Cyclohexen, α-Methylstyrol, Norbornen.

Beispielsweise sind folgende aromatische Carbaminsäureester II geeignet : Phenylcarbaminsäuremethylester ; in 2-, 3-, 5-, 6-Stellung einfach oder in 2,3-, 2,5-, 2,6-Stellung zweifach gleich oder unterschiedlich durch ein Chloratom, ein Bromatom, die Methyl-, Äthyl-, Propyl-, Isopropyl-, Butyl-, Isobutyl-gruppe substituierte Phenylcarbaminsäuremethylester ; entsprechende N-Methyl-, N-Propyl-, N-Äthyl-, N-Isopropyl-, N-Butyl-, N-Isobutyl-phenylcarbaminsäuremethylester ; homologe Äthyl-, Propyl-, Isopropyl-, Butyl-, Isobutyl-, Cyclohexyl-, Cyclopentyl-, Cycloheptyl-, Cyclooctyl-ester ; bevorzugt sind : Phenyl-, o-Tolyl-, m-Tolyl-, o-Chlorphenyl-, m-Chlorphenyl-, o-Äthylphenyl-, o-Propylphenyl-, 2-Chlor-6-methylphenyl-, 2-Chlor-5-methylphenyl-, 2,6-Dimethylphenyl- oder 2,5-Dimethylphenylcarbaminsäuremethylester und die entsprechenden Äthyl-, Propyl-, Butyl- oder Cyclohexylester.

Die Umsetzung wird im allgemeinen bei einer Temperatur von 25 bis 160 °C, vorzugsweise von 40 bis 150 °C, insbesondere 60 bis 130 °C, mit Unterdruck, Überdruck oder drucklos, vorzugsweise bei einem Druck von 1 bis 30 bar, vorzugsweise von 1 bis 20 bar, kontinuierlich oder diskontinuierlich durchgeführt. Die Verweilzeit beträgt vorzugsweise von 0,5 bis 10, insbesondere von 0,5 bis 5 Stunden, der Durchsatz vorzugsweise von 1 bis 120, insbesondere von 5 bis 50 Kilogramm Ausgangsstoff II je Kilogramm Katalysator und Stunde. Zweckmäßig verwendet man keine zusätzlichen Lösungsmittel ; gegebenenfalls kommen aber auch, z. B. zur Erniedrigung der Viskosität des Reaktionsgemisches, unter den Reaktionsbedingungen inerte Lösungsmittel in Betracht. Als Lösungsmittel kommen z. B. in Frage : aliphatische oder cycloaliphatische Kohlenwasserstoffe, z. B. Heptan, Nonan, Benzinfraktionen innerhalb

eines Siedepunktintervalls von 70 bis 190 °C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan ; Halogenkohlenwasserstoffe, insbesondere Chlorkohlenwasserstoffe, z. B. Tetrachloräthylen, 1,1,2,2-Tetrachloräthan oder 1,1,2,2-Tetrachloräthan, Dichlorpropan, Methylenchlorid, Dichlorbutan, Chloroform, Tetrachlorkohlenstoff, Tetrachloräthan, 1,1,1-Trichloräthan oder 1,1,2-Trichloräthan, Trichloräthylen, Chlorbenzol, Pentachloräthan, cis-Dichloräthylen, 1,2-Dichloräthan, 1,1-Dichloräthan ; aromatische Kohlenwasserstoffe, z. B. Benzol, Toluol, Xylole ; Tetrahydrofuran, Dioxan, Essigsäuremethylester ; und entsprechende Gemische. Zweckmäßig verwendet man das Lösungsmittel in einer Menge von 10 bis 1 000 Gewichtsprozent, vorzugsweise von 50 bis 200 Gewichtsprozent, bezogen auf Ausgangsstoff II.

Die Umsetzung wird in Gegenwart von Katalysatoren in Gestalt von anorganischen Säuren oder Sulfonsäuren, vorteilhaft mit einer Menge von 0,01 bis 0,5, insbesondere von 0,05 bis 0,3 Äquivalenten Säure, bezogen auf ein Mol Ausgangsstoff II, durchgeführt. Anstelle einbasischer Säuren können auch äquivalente Mengen mehrbasischer Säuren zur Anwendung gelangen. Beispielsweise sind folgende Säuren geeignet : Chlorwasserstoff, Schwefelsäure, Phosphorsäure ; Sulfonsäuren wie Methan-, Benzol- und p-Toluolsulfonsäure ; Borfluorwasserstoffsäure ; oder entsprechende Gemische. Die Säuren können in konzentrierter Form, im Gemisch miteinander und/oder mit einem Lösungsmittel angewendet werden.

Als Katalysatoren werden vorzugsweise organische, sulfonsaure Kationenaustauscher, vorteilhaft Harze aus sulfoniertem Polystyroldivinylbenzol, sulfonierten, vernetzten Styrolpolymeren ; sulfonsaure Phenolformaldehyd- oder Benzolformaldehydharze ; perfluorierte, sulfonierte Polystyrol-divinylbenzolharze ; perfluorierte, sulfonierte, vernetzte Polystyrolharze, oder Copolymerisate aus Tetrafluoräthylen und Vinylsulfonsäure verwendet. Vorzugsweise kommen sulfonierte Polystyroldivinylbenzolaustaucher in Frage. Die Austauscher liegen in der Säureform und nicht als Salz vor. Der Katalysator hat entsprechend seiner Anwendung vorteilhaft eine Korngröße von 5 bis 2 000, vorzugsweise von 10 bis 1 800, zweckmäßig von 20 bis 1 500 Mikrometer. Er kann sowohl makroporöse als auch gelartige Struktur besitzen. Geeignet sind z. B. Austauscherharze, wie sie unter der Bezeichnung (R)LEWASORB A-10, (R)LEWATIT SPC-118, (R)AMBERLYST 15, (R)AMBERLIT IR-120, (R)DOWEX 50, (R)LEWATIT S-100, (R)NALCITE HCR, (R)PERMUTIT RS, (R)WOLFATIT KPS-200 im Handel erhältlich sind. Man entwässert sie zweckmäßig vor der Verwendung in üblicher Weise, z. B. durch Erhitzen auf 100 °C bis 110 °C im Vakuum. Die entwässerung kann jedoch auch durch Verdrängen mit hydrophilen organischen Flüssigkeiten und anschließendem Erhitzen auf 100 °C bei vermindertem Druck oder durch azeotrope Destillation mit einer organischen Flüssigkeit erfolgen.

Der Katalysator in Gestalt eines Ionenaustauschers kann in beliebiger diskontinuierlicher oder kontinuierlicher Arbeitsweise, z. B. in Gestalt eines Festbetts, verwendet werden. Er befindet sich ebenfalls vorteilhaft während der Umsetzung in Suspension, in der Regel im sich bildenden Reaktionsgemisch. Zweckmäßig legt man einen Anteil an flüssigem Carbaminsäureester II und Olefin III und gegebenenfalls Lösungsmittel vor und suspendiert den Katalysator in der Flüssigkeit unter guter Durchmischung. Man wählt vorteilhaft die Menge an vorgelegtem Carbaminsäureester II oder Ausgangsgemisch und/oder organischem Lösungsmittel so, daß der Katalysator in Gestalt eines Ionenaustauschers im sich bildenden Reaktionsgemisch in einer Menge von 0,3 bis 30, vorzugsweise 1 bis 20 Gewichtsprozent, bezogen auf das Gewicht des gesamten flüssigen Gemischs im Reaktionsraum, suspendiert ist. Bevorzugt ist eine Menge von 1 bis 70, insbesondere von 10 bis 50 Gewichtsprozent Ionenaustauscher, bezogen auf Carbaminsäureester. Zweckmäßig wird das Reaktionsgemisch während der gesamten Umsetzung durchmischt, vorzugsweise mit einer Rührgeschwindigkeit von mindetens 100, zweckmäßig von 200 bis 2 000, insbesondere von 300 bis 1 000 Umdrehungen pro Minute. Bei Durchmischungseinrichtungen ohne Rührwerk, z. B. auch bei Durchmischung mittels Inertgas wie Stickstoff, werden solche bevorzugt, die vorgenannter Rührgeschwindigkeit entsprechende Scherenergie einbringen. Auf dieses Weise wird eine feindisperse Suspension erzielt.

Die Reaktion kann wie folgt durchgeführt werden : Ein Gemisch von Ausgangsstoff II und III, Katalysator und gegebenenfalls Lösungsmittel wird während der Reaktionszeit bei der Reaktionstemperatur gehalten. Nach Abtrennung des Katalysators kann man den Endstoff in üblicher Weise, z. B. durch Filtration und Destillation, isolieren.

Die Reaktion kann im Falle der Verwendung von Ionenaustauschern als Katalysatoren und im kontinuierlichen Betrieb im Festbett oder vorteilhaft wie folgt durchgeführt werden : Ein flüssiges Gemisch von Carbaminsäureester, Olefin, gegebenenfalls zusammen mit Lösungsmittel, wird bei der Reaktionstemperatur und dem Reaktionsdruck durch eine Suspension des Katalysators im Ausgangsgemisch bzw. Reaktionsgemisch geleitet und filtriert. Dann wird aus dem Reaktionsgemisch der Endstoff in üblicher Weise, z. B. durch Destillation, abgetrennt. Die Filtrierung erfolgt zweckmäßig vor Austritt der Suspension aus dem Reaktor. Als Filter kommen säurefeste Filtertücher, Drahtnetzfilter, Sintermetallfilter, sofern die Maschenweiten bzw. Porendurchmesser kleiner als die Katalysatorpartikel sind, in Betracht.

Die nach dem Verfahren der Erfindung herstellbaren aromatischen Carbaminsäureester I sind Wirkstoffe und wertvolle Ausgangsstoffe für die Herstellung von Farbstoffen, Schädlingsbekämpfungsmitteln und Pharmazeutika. Durch Hydrolyse der Carbaminsäureester (Houben-Weyl, Band 11/1, Seiten 448 bis 952) können die entsprechenden Aniline hergestellt werden, die ebenfalls wichtige Ausgangsprodukte bei der Synthese von Wirkstoffen darstellen. Bezüglich der Verwendung wird auf die vorgenannten

Veröffentlichungen, OE-PS 183 416 und Ullmanns Encyklopädie der technischen Chemie, Band 5, Seiten 73 bis 76, verwiesen.

Die in den folgenden Beispielen aufgeführten Teile bedeuten Gewichtsteile. Sie verhalten sich zu den Volumenteilen wie Kilogramm zu Liter.

## Beispiel 1

In einem Rührreaktor wird eine Suspension von 165 Teilen N-Phenylcarbaminsäureäthylester und 50 Teilen Austauscherharz mit einer Rührgeschwindigkeit von 300 Umdrehungen pro Minute bei 120 °C hergestellt und innerhalb von 2 Stunden 100 Teile Isobuten zugepumpt. Durch das Zupumpen von Isobutylen wird ein Druck von 2 bar eingestellt. Das Austauscherharz ist ein sulfoniertes, aus Styrol und Divinylbenzol copolymerisiertes Harz, das vor der Verwendung während 24 Stunden bei 100 °C im Vakuum entwässert wird ; es besitzt eine Korngröße von 0,5 bis 1,5 Millimetern. Nun wird die Suspension im Reaktor ständig mit 500 Umdrehungen pro Minute gerührt. Nach 2 Stunden bei 120 °C wird die Suspension filtriert und einer fraktionierenden Destillation zugeführt. Man erhält 170 Teile (77 % der Theorie, bezogen auf den N-Phenylcarbaminsäureäthylester) N-(4-tert.-Butylphenyl)-carbaminsäureäthylester vom Kp. 125 °C-127 °C (0,2 mbar). Der Umsatz beträgt 80 Prozent (bezogen auf den Carbaminsäureester II).

## Beispiel 2

In einem Rührreaktor wird eine Suspension von 165 Teilen N-Phenylcarbaminsäureäthylester und 30 Teilen Austauscherharz mit einer Rührgeschwindigkeit von 300 Umdrehungen pro Minute bei 100 °C hergestellt und innerhalb von einer Stunde 130 Teile Styrol bei 1 bar zugeführt. Das Austauscherharz ist ein sulfoniertes, aus Styrol und Divinylbenzol copolymerisiertes Harz, das vor der Verwendung während 24 Stunden bei 100 °C im Vakuum entwässert wird ; es hat Gelstruktur und besitzt eine Korngröße von 20 bis 150 Mikrometern. Nun wird die Suspension im Reaktor bei 100 °C eine Stunde mit 300 Umdrehungen pro Minute gerührt. Anschließend wird der Katalysator abgetrennt, nicht umgesetzter Ausgangsstoff im Vakuum abdestilliert und der Rückstand aus Cyclohexan umkristallisiert. Man erhält 240 Teile (89 % der Theorie, bezogen auf den Carbaminsäureester) N-(4-(α-Methylbenzyl)-phenyl)-carbaminsäureäthylester vom Fp 78 bis 80 °C. Der Umsatz beträgt 90 Prozent.

## Beispiel 3

In einem Rührreaktor wird eine Suspension von 165 Teilen N-Phenylcarbaminsäureäthylester und 50 Teilen Austauscherharz mit einer Rührgeschwindigkeit von 300 Umdrehungen pro Minute bei 120 °C hergestellt und innerhalb von einer Stunde bei 2 bar 100 Teile 2-Methylbuten-2 zugeführt. Das Austauscherharz ist ein sulfoniertes, aus Styrol und Divinylbenzol copolymerisiertes Harz, das vor der Verwendung während 24 Stunden bei 100 °C im Vakuum entwässert wird ; es hat Gelstruktur und besitzt eine Korngröße von 20 bis 150 Mikrometern. Nun wird die Suspension im Reaktor 2 Stunden mit 300 Umdrehungen pro Minute bei 120 °C gerührt. Anschließend wird der Katalysator abfiltriert und im Vakuum destilliert. Man erhält 195 Teile (82 % der Theorie, bezogen auf den Carbaminsäureester II) N-(4-tert.-Amylphenyl-)carbaminsäureäthylester vom Kp 145 bis 147 °C (0,3 mbar). Der Umsatz beträgt 84 Prozent (bezogen auf den Carbaminsäureester II).

## Beispiel 4

In einem Rührreaktor wird ein Gemisch aus 165 Teilen N-Phenylcarbaminsäureäthylester und 10 Teilen Schwefelsäure (96-prozentig) unter Rühren auf 70 °C erhitzt und innerhalb von zwei Stunden 104 Teile Styrol zugeführt. Nach Beendigung der Styrolzugabe wird das Gemisch noch zwei Stunden bei 70 °C gerührt. Anschließend wird das Gemisch mit 100 Teilen Toluol verdünnt, mit wäßriger Natriumcarbonatlösung neutralisiert und im Vakuum destilliert. Nach der Umkristallisation aus Cyclohexan erhält man 185 Teile (69 % der Theorie, bezogen auf den Carbaminsäureester II) N-(4-(α-Methylbenzyl)-phenyl)-carbaminsäureäthylester vom Fp 78 bis 80 °C. Der Umsatz beträgt 75 Prozent.

## Beispiel 5

In einem Rührautoklaven wird ein Gemisch aus 165 Teilen N-Phenylcarbaminsäureäthylester und 10 Gramm p-Toluolsulfonsäure unter Rühren auf 100 °C erhitzt und innerhalb von zwei Stunden 112 Teile Isobuten zugepumpt. Nach Beendigung der Isobutenzugabe wird das Gemisch noch zwei Stunden bei 100 °C gerührt. Anschließend wird das Gemisch mit 100 Teilen Toluol verdünnt, mit wäßriger Natriumcarbonatlösung extrahiert und der Endstoff im Vakuum destilliert. Man erhält 169 Teile (72 % der Theorie, bezogen auf den Carbaminsäureester II) N-(4-tert.-Butylphenyl)-carbaminsäureäthylester vom Kp 125 bis 127 °C (0,2 mbar). Der Umsatz beträgt 76 Prozent.

**Anspruch**

Verfahren zur Herstellung von p-substituierten, aromatischen Carbaminsäureestern der Formel

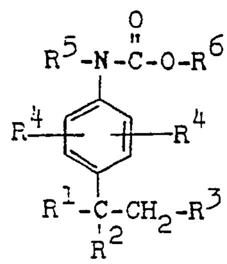

I

worin die einzelnen Reste $R^1$, $R^2$, $R^3$, $R^4$, $R^5$ und $R^6$ gleich oder verschieden sein können und jeweils einen aliphatischen Rest bedeuten, $R^1$, $R^2$ und $R^3$ auch jeweils einen cycloaliphatischen, araliphatischen oder aromatischen Rest bezeichnen, jeweils 2 der Reste $R^1$, $R^2$ und $R^3$ auch zusammen mit den benachbarten Kohlenstoffatomen für Glieder eines alicyclischen Ringes oder $R^1$, $R^2$ und $R^3$ auch zusammen mit den benachbarten Kohlenstoffatomen für einen bicyclischen Rest stehen, $R^2$, $R^3$ und $R^5$ auch jeweils ein Wasserstoffatom bezeichnen, $R^4$ auch ein Wasserstoffatom oder ein Halogenatom bedeutet, $R^6$ auch einen cycloaliphatischen Rest bezeichnet, dadurch gekennzeichnet, daß man aromatische Carbaminsäureester der Formel

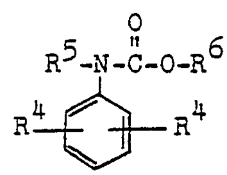

II

worin $R^4$, $R^5$ und $R^6$ die vorgenannte Bedeutung besitzen, mit Olefinen der Formel

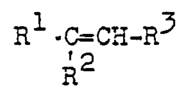

III

worin $R^1$, $R^2$ und $R^3$ die vorgenannte Bedeutung besitzen, in Gegenwart von anorganischen Säuren oder Sulfonsäuren umsetzt.

**Claim**

A process for the preparation of a p-substituted, aromatic carbamic acid ester of the formula

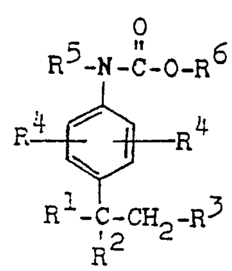

I

where the individual radicals $R^1$, $R^2$, $R^3$, $R^4$, $R^5$ and $R^6$ may be identical or different and each is an aliphatic radical, $R^1$, $R^2$ and $R^3$ may also each be a cycloaliphatic, araliphatic or aromatic radical, any 2 of the radicals $R^1$, $R^2$ and $R^3$ may also, conjointly with the adjacent carbon atoms, be members of an

alicyclic ring, $R^1$, $R^2$ and $R^3$ conjointly with the adjacent carbon atoms may also be a bicyclic radical, $R^2$, $R^3$ and $R^5$ may also each be hydrogen, $R^4$ may also be hydrogen or halogen and $R^6$ may also be a cycloaliphatic radical, wherein an aromatic carbamic acid ester of the formula

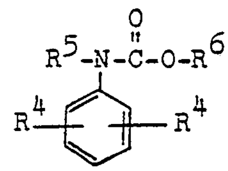

II

where $R^4$, $R^5$ and $R^6$ have the above meanings, is reacted with an olefin of the formula

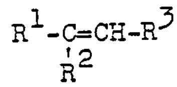

III

where $R^1$, $R^2$ and $R^3$ have the above meanings, in the presence of an inorganic acid or sulfonic acid.

**Revendication**

Procédé pour la préparation de carbamates aromatiques p-substitués de formule

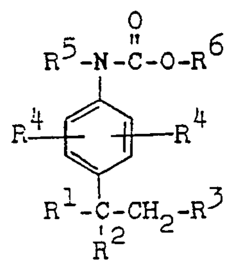

I

dans laquelle les différents radicaux $R^1$, $R^2$, $R^3$, $R^4$, $R^5$ et $R^6$ peuvent être semblables ou différents et désignent chacun un radical aliphatique, $R^1$, $R^2$ et $R^3$ pouvant aussi représenter chacun un radical cycloaliphatique, araliphatique ou aromatique, deux des radicaux $R^1$, $R^2$ et $R^3$ pouvant aussi constituer, avec les atomes de carbone voisins, des termes d'un noyau alicyclique ou $R^1$, $R^2$ et $R^3$ pouvant former, avec les atomes de carbone voisins, un radical bicyclique, $R^2$, $R^3$ et $R^5$ pouvant aussi désigner chacun un atome d'hydrogène, $R^4$ pouvant également représenter un atome d'hydrogène ou un atome d'halogène, $R^6$ pouvant être aussi un radical cycloaliphatique, caractérisé en ce qu'on fait réagir des carbamates aromatiques de formule

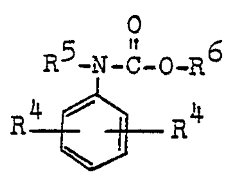

II

dans laquelle $R^4$, $R^5$ et $R^6$ ont les significations données ci-dessus, avec des oléfines de formule

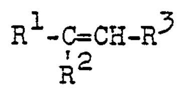

III

dans laquelle $R^1$, $R^2$ et $R^3$ ont les significations données ci-dessus, en présence d'acides inorganiques ou d'acides sulfoniques.